# EUROPEAN PATENT APPLICATION

(11) **EP 3 949 920 A1**
(43) Date of publication of application: **09.02.2022**
(21) Application number: 20810575.9
(22) Date of filing: 25.03.2020
(51) Int. Cl.: A61F 13/15, A61F 13/49, A61F 13/496, A61F 13/51

(54) **PANTS-TYPE DISPOSABLE DIAPER AND METHOD FOR MANUFACTURING PANTS-TYPE DISPOSABLE DIAPER**

(30) Priority: 20.05.2019 JP 2019094756
(71) Applicant: Unicharm Corporation, Shikokuchuo-shi, Ehime 799-0111 (JP)
(72) Inventor: SASAYAMA, Kenichi, Kanonji-shi, Kagawa 769-1602 (JP); MURAKAMI, Kei, Kanonji-shi, Kagawa 769-1602 (JP); NAGAYAMA, Yui, Kanonji-shi, Kagawa 769-1602 (JP); KONDO, Daiki, Kanonji-shi, Kagawa 769-1602 (JP)
(74) Representative: Staeger & Sperling Partnerschaftsgesellschaft mbB
(86) International application number: PCT/JP2020/013443
(87) International publication number: WO 2020/235212

(57) **Abstract**

This pants-type disposable diaper (1) is provided with a belt part (20) and an absorbent body (20), wherein: the belt part (20) has an exterior sheet (214) and an elastic film (60); the exterior sheet (214) and the elastic film (60) are jointed to each other by a plurality of joining parts (60); the elastic film (60) has a stretchable layer (61) stretchable in the left-right direction, and a less-stretchable layer (62) laminated on the stretchable layer (61) and having a lower stretchability than that of the stretchable layer (61); the less-stretchable layer (62) continues in one joining part (60) among the plurality of joining parts (60); and the stretchable layer (61) and the less-stretchable layer (62) are peeled off from at least a portion of the outer edge section of the one joining part (60).

## Description

### FIELD

The present invention relates to an underpants-shaped disposable diaper and a method for manufacturing an underpants-shaped disposable diaper.

### BACKGROUND

Underpants-shaped disposable diapers have been known in which a sheet member formed by overlaying a nonwoven fabric and an elastic film is used to enhance a surface-to-surface fitting property. For example, Patent Literature 1 discloses an absorbent article in which an elastic film is placed between a first sheet layer (nonwoven fabric) and a second sheet layer (nonwoven fabric), and in which, by forming through holes that penetrate the elastic film, these overlaid sheet members are joined to each other.

### [CITATION LIST]

### [PATENT LITERATURE]

Patent Literature 1: Japanese Patent Application Publication No. 2016-189826

### SUMMARY

### [TECHNICAL PROBLEM]

In the disposable diaper of Patent Literature 1, due to the through holes provided in the elastic film, high breathability can be embodied at a joining portion of the sheet members. However, in the configuration in which the through holes are provided in the elastic film as in Patent Literature 1, there is a risk that it is partially less likely for the stretching/contracting force to act, since the elastic film discontinues at positions between the adjacent through holes (joining portions) that are adjacent in the stretching/contracting direction. In the case where such sheet members are used in a disposable diaper, there is a risk that the stretching/contracting force exhibited by the elastic film becomes uneven, impairing the fitting property to the wearer's body.

On the other hand, in the case where the through holes are not formed in the joining portion where the elastic film and the nonwoven fabric are joined, since the stiffness of the sheet members increased by the joining portion is not relaxed by the through holes, there is a risk that the stiffness of the entire sheet members increases, making insufficient the followability and fitting property to the wearer's body.

The present invention was achieved in light of problems that described above and an aspect of the present invention is to provide an underpants-shaped disposable diaper including an elastic film and having good fitting property.

### [SOLUTION TO PROBLEM]

A main aspect of the present invention for achieving the above-described aspect is an underpants-shaped disposable diaper having a vertical direction, a lateral direction, and a front-back direction that intersect each other, the disposable diaper including: a belt portion; and an absorbent main body, the belt portion having an exterior sheet and an elastic film, the elastic film being stretchable at least in the lateral direction, the exterior sheet and the elastic film being joined to each other by a plurality of joining portions, the elastic film having a stretchable layer and a low stretchable layer, the stretchable layer being a layer that stretches and contracts in the lateral direction, the low stretchable layer being a layer that is overlaid on the stretchable layer and has lower stretchability than the stretchable layer, in a certain joining portion of the plurality of joining portions, the low stretchable layer being continuous, in at least a part of an outer edge portion of the certain joining portion, the stretchable layer and the low stretchable layer being peeled off.
Features of the present invention other than the above will become clear by reading the description of the present specification with reference to the accompanying drawings.

### [ADVANTAGEOUS EFFECT OF THE INVENTION]

According to the present invention, it is possible to provide an underpants-shaped disposable diaper including an elastic film and having good fitting property.

### BRIEF DESCRIPTION OF DRAWINGS

FIG. 1 is a schematic perspective view of an underpants-shaped disposable diaper 1.
FIG. 2 is a schematic plan view of the diaper 1 in an unfolded and stretched state.
FIG. 3 is a schematic cross-sectional view taken along a line I-I in FIG. 2.
FIG. 4 is an enlarged schematic cross-sectional view of a portion in which a joining portion 50 is not formed, in a thickness-direction cross section of a belt portion 21.
FIG. 5 is an enlarged schematic cross-sectional view of a portion in which the joining portion 50 is formed, in the thickness-direction cross section of a belt portion 21.
FIG. 6 is a photograph of an actual cross-sectional structure of the belt portion 21 in the thickness direction.
FIG. 7 is a photograph of a region A of FIG. 6 in an enlarged manner.
FIG. 8 is a photograph of a cross-sectional structure of the belt portion 21 in the thickness direction in the case where a part of an elastomer layer 61 is discontinuous.
FIG. 9 is a flow chart of steps in manufacturing the diaper 1.
FIG. 10 is a schematic diagram illustrating a manufacturing apparatus 100 for manufacturing the diaper 1.
FIG. 11 is a diagram illustrating the arrangement of a plurality of protrusion portions 122a provided on an outer circumferential surface of an anvil roll 122.

### DESCRIPTION OF EMBODIMENTS

At least following matters will become clear with description of this specification and attached drawings.
An underpants-shaped disposable diaper having a vertical direction, a lateral direction, and a front-back direction that intersect each other, the disposable diaper including: a belt portion; and an absorbent main body, the belt portion having an exterior sheet and an elastic film, the elastic film being stretchable at least in the lateral direction, the exterior sheet and the elastic film being joined to each other by a plurality of joining portions, the elastic film having a stretchable layer and a low stretchable layer, the stretchable layer being a layer that stretches and contracts in the lateral direction, the low stretchable layer being a layer that is overlaid on the stretchable layer and has lower stretchability than the stretchable layer, in a certain joining portion of the plurality of joining portions, the low stretchable layer being continuous, in at least a part of an outer edge portion of the certain joining portion, the stretchable layer and the low stretchable layer being peeled off.

According to the above-described underpants-shaped disposable diaper, in the joining portion of the elastic film and the exterior sheet, at least the low stretchable layer is continuous in the elastic film. This can suppress decrease of the stretchability of the belt portion (elastic film). In addition, in the outer edge portion of the joining portion, there is a portion in which the stretchable layer and the low stretchable layer are peeled off, and this makes it easier for the elastic film to follow the movement of the wearer's body, making it possible to enhance the texture. Thus, good fitting property when the diaper is put on can be embodied.

In the underpants-shaped disposable diaper, it is desirable that in a natural state, a thickness of the belt portion in a central portion of the joining portion is smaller than a thickness of the elastic film in a region where the joining portion is not provided.

According to the above-described underpants-shaped disposable diaper, the elastic film does not exhibit stretchability in the central portion of the joining portion, and therefore the joining state between the sheet members is stabilized, making it easier to maintain the joining strength. Thus, the exterior sheet easily follows the stretching and contraction of the elastic film, maintaining good stretchability in the entire belt portion, making it easier to fit the belt portion to the wearer's body.

In the underpants-shaped disposable diaper, it is desirable that the exterior sheet has a skin-side sheet that is arranged on a skin side with respect to the elastic film, and a non-skin-side sheet that is arranged on a non-skin side with respect to the elastic film, and it is desirable that the elastic film, the skin-side sheet, and the non-skin-side sheet are joined by the joining portion.

According to the above-described underpants-shaped disposable diaper, the elastic film is fixed so as to be sandwiched between the exterior sheets from two sides in a thickness direction, and therefore the joining force between the exterior sheet and the elastic film can increase compared with the case of fixing on only one side. Thus, even in the case where the elastic film stretches and contracts, the exterior sheet and the elastic film are less likely to peel off, making it possible to improve the stretchability of the entire belt portion.

In the underpants-shaped disposable diaper, it is desirable that the low stretchable layer is formed of a predetermined resin material, and that the low stretchable layer is overlaid on each of two sides of the stretchable layer in a thickness direction.

According to the above-described underpants-shaped disposable diaper, when joining the elastic film and the exterior sheet, it is possible to more firmly join them by melting and joining to each other a resin constituting the low stretchable layer and a resin constituting a nonwoven fabric of the exterior sheet. Thus, even in the case where the belt portion greatly stretches and contracts, the elastic film and the exterior sheet are less likely to peel off, making it easier for the belt portion to follow the movement of the wearer's body.

In the underpants-shaped disposable diaper, it is desirable that the low stretchable layer is formed of a polyolefin resin, and
that the exterior sheet is formed of a nonwoven fabric that has fibers containing the polyolefin resin.

According to the above-described underpants-shaped disposable diaper, when joining the low stretchable layer and the exterior sheet that are overlaid in the thickness direction, the polyolefin resins are easily welded to each other, making it possible to more firmly join the low stretchable layer and the exterior sheet. Thus, the belt portion more easily follows the movement of the wearer's body, and the fitting property of the diaper is further enhanced.

In the underpants-shaped disposable diaper, it is desirable that in an outer extension portion of the joining portion, the stretchable layer has a portion that is discontinuous.

According to the above-described underpants-shaped disposable diaper, the presence of the discontinuous portion in the stretchable layer increases the flexibility of the elastic film in the discontinuous portion, making it easier for the elastic film to flexibly deform at the outer edge portion of the highly stiff joining portion. This increases the followability of the belt portion to the movement of the wearer's body when the diaper is put on, and makes the wearer to be less likely to feel hard touch in the joining portion.

In the underpants-shaped disposable diaper, it is desirable that in the belt portion, a plurality of the elastic films are arranged side-by-side with spaces in the vertical direction, the plurality of elastic films each having a band-like shape and being laterally elongated.

According to the above-described underpants-shaped disposable diaper, by providing a predetermined space between two elastic films adjacent in the vertical direction, the space makes it possible to easily secure the breathability of the belt portion. This can make the wearer be less likely to feel discomfort when the diaper is put on.

In the underpants-shaped disposable diaper, it is desirable that the belt portion has a stretchable nonwoven fabric that is stretchable at least in the lateral direction.

According to the above-described underpants-shaped disposable diaper, by providing the stretchable nonwoven fabric that has a large number of gaps between the entangled fibers, it is possible to enhance the stretchability and breathability of the belt portion. This makes it possible to further enhance the fitting property of the belt portion when the diaper is put on.

There is clarified a method for manufacturing an underpants-shaped disposable diaper including: a transport step of transporting an elastic-film continuous body and an exterior-sheet continuous body at a predetermined transport speed in a direction of transport; a joining step of joining the elastic-film continuous body and the exterior-sheet continuous body, forming a belt portion, the elastic-film continuous body and the exterior-sheet continuous body being transported in a thickness direction; and an absorbent-main-body attachment step of attaching an absorbent main body to the belt portion, in the transport step, stretching the elastic-film continuous body in the direction of transport at a predetermined stretching ratio, subsequently loosening the elastic-film continuous body so that the elastic-film continuous body has a stretching ratio lower than the predetermined stretching ratio, in the joining step, joining the elastic-film continuous body to the exterior-sheet continuous body.

According to the above-described method for manufacturing an underpants-shaped disposable diaper, the low stretchable layer of the elastic film is greatly stretched in advance in the transport step, and therefore it is possible to provide sufficient allowance in the stretchability of the elastic film. This makes it possible to prevent the low stretchable layer from being torn or punctured in the manufacturing step of the diaper. Thus, the elastic film easily follows the movement of the wearer's body, and a diaper having good fitting property can be embodied.

In the method for manufacturing an underpants-shaped disposable diaper, it is desirable that, in the joining step, the elastic-film continuous body and the exterior-sheet continuous body are joined by applying ultrasonic vibration using an ultrasonic horn and an anvil, the ultrasonic horn being arranged on a thickness-direction one side of the elastic-film continuous body and the exterior-sheet continuous body, the anvil being arranged on a thickness-direction other side, and that the ultrasonic horn and the anvil perform the joining while moving in the direction of transport at a speed equal to a transport speed at which the elastic-film continuous body and the exterior-sheet continuous body are transported.

According to the above-described method for manufacturing an underpants-shaped disposable diaper, in the joining step, ultrasonic welding is performed while the ultrasonic horn and the anvil move in the same direction at the same speed as the transport speed of the materials, and this makes it possible to accurately form the joining portion without hindering the transport of the materials. Thus, at the time of forming the joining portion, the low stretchable layer of the elastic film is less likely to be torn, making it easier to maintain a continuous state. Thus, the fitting property of the belt portion is easily enhanced.

In the method for manufacturing an underpants-shaped disposable diaper, it is desirable that the ultrasonic horn is provided on an outer circumferential surface of an ultrasonic roll, the ultrasonic roll being a cylindrical roll that has a rotation axis extending in a direction orthogonal to the direction of transport, that the anvil is provided on an outer circumferential surface of an anvil roll, that the anvil protrudes outward in a radial direction from the outer circumferential surface of the anvil roll, the anvil roll being a cylindrical roll that has a rotation axis extending in a direction orthogonal to the direction of transport, and that, in the joining step, a number of sheet members arranged between the elastic film and the anvil roll is larger than a number of sheet members arranged between the elastic film and the ultrasonic roll.

According to the above-described method for manufacturing an underpants-shaped disposable diaper, by increasing the number of sheet members arranged between the elastic film and the protrusion portions of the anvil roll, it is possible to easily relax the impact that acts on the elastic film from the protrusion portions when ultrasonic welding is performed. Thus, the elastic film is less likely to be torn, making it easier to maintain a state in which the low stretchable layer is continuous at the joining portion. Thus, the fitting property of the belt portion can be enhanced.

In the method for manufacturing an underpants-shaped disposable diaper, it is desirable that an area ratio of the anvil provided on the outer circumferential surface of the anvil roll is equal to or more than 1% and equal to or less than 9%.

According to the above-described method for manufacturing an underpants-shaped disposable diaper, it is possible to suppress the deterioration of the stretchability of the elastic film and of the texture of the belt portion while maintaining the sufficient joining strength between the elastic film and the exterior sheet.

### Embodiment

As an underpants-shaped disposable diaper according to the present embodiment, an underpants-shaped disposable diaper 1 for adults (hereinafter, also simply referred to as a "diaper 1") will be described as an example. However, the present invention is not limited thereto, and the underpants-shaped disposable diaper according to the present invention is also applicable to, for example, disposable diapers for children (for infants), sanitary shorts, and the like.

### Basic Configuration of Diaper 1

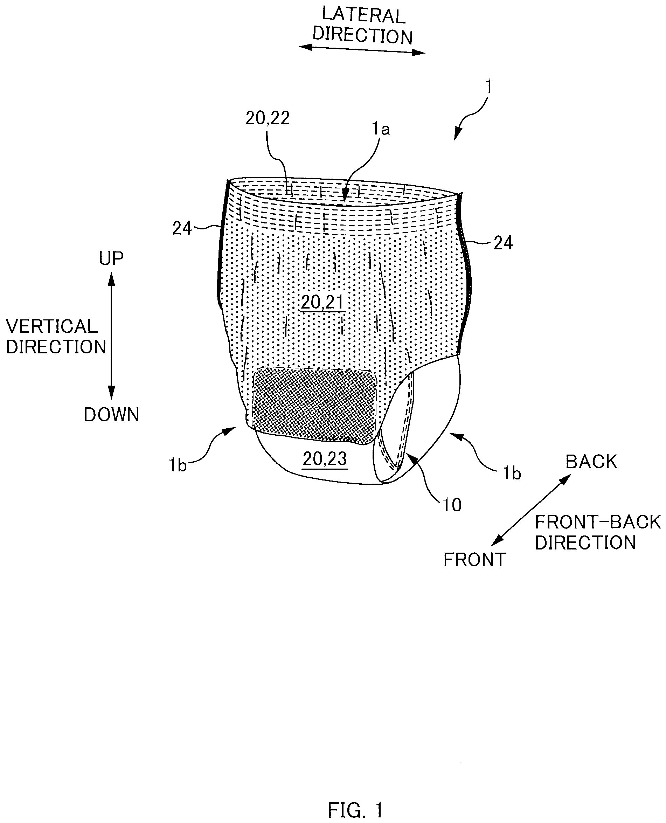

FIG. 1 is a schematic perspective view of an underpants-shaped disposable diaper 1.

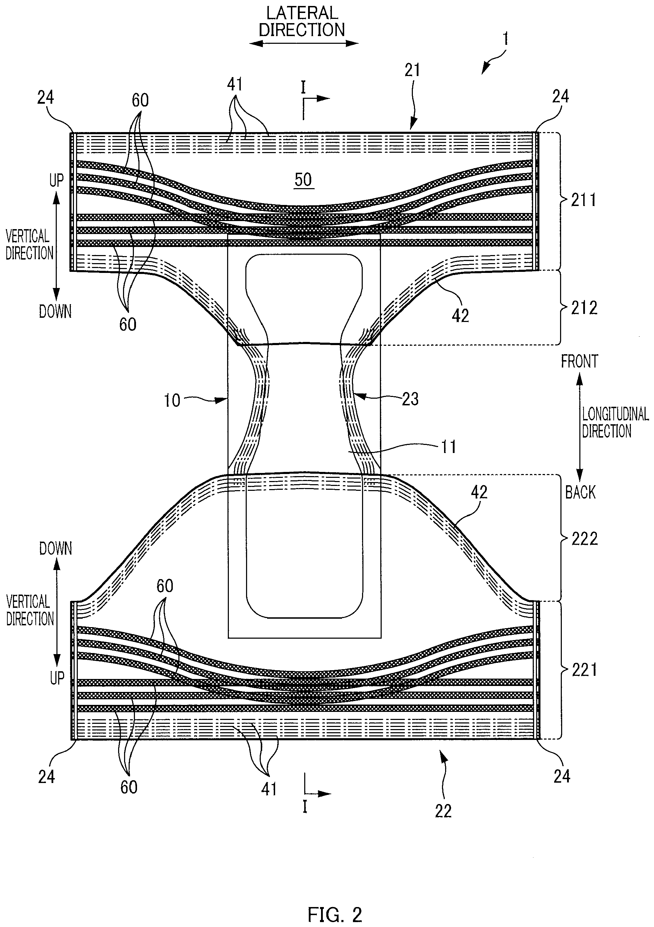

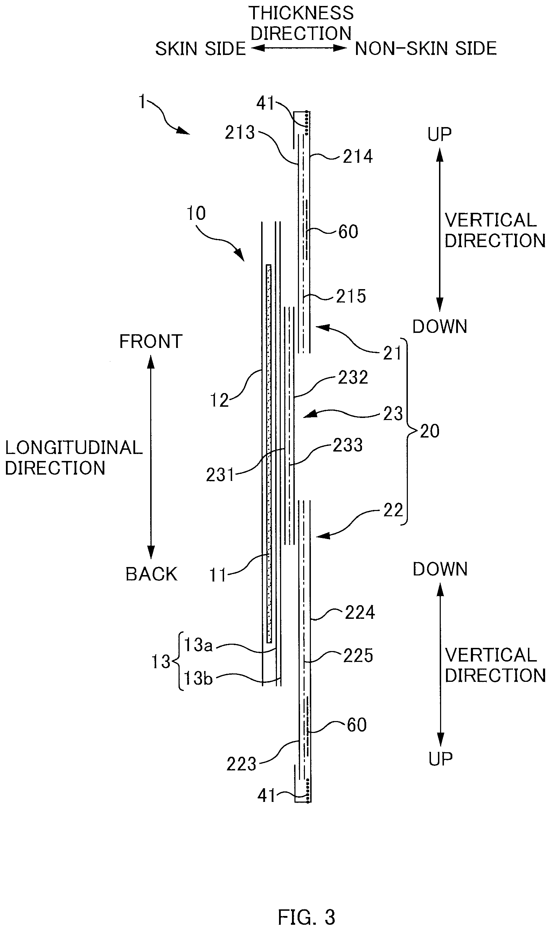

FIG. 2 is a schematic plan view of the diaper 1 in an unfolded and stretched state. FIG. 3 is a schematic cross-sectional view taken along a line I-I (at the lateral center) in FIG. 2.

The diaper 1 has a vertical direction, a lateral direction, and a front-back direction that intersect with each other, and has a thickness direction in which constituent members are overlaid as shown in FIG. 3. In the vertical direction, the side corresponding to the wearer's trunk is the upper side, and the side corresponding to the wearer's crotch is the lower side. In the front-back direction, the side corresponding to the wearer's stomach is the front side, and the side corresponding to the wearer's back is the back side. In the thickness direction, the side that comes into contact with the wearer is the skin side, and the opposite side is the non-skin side.

The diaper 1 includes an absorbent main body 10 and an exterior body 20 that is positioned on a non-skin side of the absorbent main body 10. The exterior body 20 includes: a front belt portion 21 that is arranged on the wearer's stomach side; a back belt portion 22 that is arranged on the wearer's back side; and a crotch portion 23 that connects the front belt portion and the back belt portion.

In the diaper 1 of the present embodiment, the exterior body 20 is formed of three members (the front belt portion 21, the back belt portion 22, and the crotch portion 23), but the configuration is not limited thereto. For example, the front belt portion 21, the back belt portion 22, and the crotch portion 23 may be formed of one continuous member. In addition, there may also be a configuration in which the crotch portion 23 is not included.

In the diaper 1 in an unfolded state shown in FIG. 2, the front belt portion 21 is positioned on the longitudinal one end side of the absorbent main body 10, and the back belt portion 22 is positioned on the longitudinal other end side of the absorbent main body 10. In the diaper 1 in the unfolded state, the absorbent main body 10 is folded one time at the substantially center in the longitudinal direction. Then, two lateral side portions of the front belt portion 21 and two lateral side portions of the back belt portion 22 are locked by means such as welding, and a pair of locking portions 24 are formed. Thus, an underpants-shaped diaper shown in FIG. 1 is formed. That is, the longitudinal direction of the absorbent main body 10 is in the vertical direction of the diaper 1, the front belt portion 21 and the back belt portion 22 are connected in an annular shape, a waist opening 1a is formed at an upper end thereof, and a pair of leg openings 1b and 1b are formed on two lateral sides of the diaper 1.

As shown in FIG. 3, the absorbent main body 10 has an absorbent core 11, a liquid-permeable top sheet 12 that is positioned on the skin side of the absorbent core 11, and a back sheet 13 that is positioned on the non-skin side of the absorbent core 11. The back sheet 13 of the present embodiment has a two-layer structure including a liquid-impermeable sheet 13a and a hydrophobic liquid-permeable sheet 13b (for example, a hydrophobic nonwoven fabric) arranged on the non-skin side of the sheet 13a.

The absorbent core 11 is a member that absorbs and holds excreted fluid such as urine, and is formed of, for example, a liquid absorbent fiber (e.g., a pulp fiber) containing a superabsorbent polymer (SAP). Although not shown, an outer circumferential surface of the absorbent core 11 may be covered with a liquid-permeable sheet such as tissue paper or a nonwoven fabric.

As shown in FIG. 2, in the vertical direction, the front belt portion 21 and the back belt portion 22 respectively have waist regions 211 and 221 that are portions that overlap the locking portions 24, and crotch regions 212 and 222 that are arranged below the corresponding waist regions 211 and 221.

The crotch regions 212 and 222 are substantially trapezoidal, and the lateral width (length in the lateral direction) decreases toward the lower side. Compared with the front belt portion 21, the crotch region 222 of the back belt portion 22 is larger so as to be able to cover the wearer's buttocks.

As shown in FIG. 3, the front belt portion 21, the back belt portion 22, and the crotch portion 23 respectively have skin-side sheets 213, 223, and 231, non-skin-side sheets 214, 224, and 232, and stretchable nonwoven fabrics 215, 225, and 233, the stretchable nonwoven fabrics being positioned respectively between the skin-side sheets and the non-skin-side sheets. Sheet members overlaid in the thickness direction are joined to each other by a plurality of joining portions 50, the joining portions 50 being intermittently arranged spacing from each other. The configuration of the crotch portion 23 is not particularly limited. There may also be a configuration in which, for example, the skin-side sheet 231 is not included and the stretchable nonwoven fabric 233 is arranged between the absorbent main body 10 and the non-skin-side sheet 232.

In the present embodiment, the skin-side sheets 213 and 223 and the non-skin-side sheets 214 and 224 correspond to exterior sheets that constitute the exterior of the belt portions 21 and 22. Examples of the non-skin-side sheets 214 and 224 and the skin-side sheets 213 and 223 include flexible sheets formed of spunbond nonwoven fabric, SMS nonwoven fabric, or the like, made of fibers containing polypropylene (PP), polyethylene (PE), or the like (a polyolefin-based resin).

Examples of the stretchable nonwoven fabrics 215 and 225 include nonwoven fabrics that is obtained by stretchable fibers and non-stretchable fibers being subject to an appropriate stretching processing such as gear stretching, the stretchable fibers being a polyurethane-based elastomer that is a kind of an elastic thermoplastic elastomer, the non-stretchable fibers being polypropylene (PP) of a polyolefin-based resin that is a kind of a non-elastic thermoplastic resin. The stretching treatment may be performed in mutually orthogonal directions (the lateral direction and the vertical direction of the diaper 1), or may be performed only in a predetermined direction (the lateral direction of the diaper 1). It should be noted that, in the case where the stretching treatment is performed only in the predetermined direction, stretchability is exhibited in the predetermined direction. But, it is not necessary the case that all of the fibers are oriented in the predetermined direction, and therefore, stretchability is exhibited also in a direction orthogonal to the predetermined direction.

In addition, in the diaper 1 of the present embodiment, as shown in FIG. 2, the elastic films 60 are continuously arranged in the circumferential direction (corresponding to the lateral direction in FIG. 2) across the waist region 211 of the front belt portion 21 and the waist region 221 of the back belt portion 22. The elastic film 60 is a stretchable sheet that is stretchable at least in the circumferential direction (the lateral direction, that is, the direction around the wearer's waist), and is overlaid on the stretchable nonwoven fabrics 215 and 225 as shown in FIG. 3. Therefore, in the region in which the elastic film 60 is arranged, the tightening pressure is high compared with the surrounding region, and the region strongly comes close contact with the wearer. As described above, by arranging the elastic film 60 that is in close and continuous contact with the wearer's waist only in a part of the front belt portion 21 and the back belt portion 22, it is possible to suppress the excessive tightening of the wearer's waist while suppressing the positional deviation of the diaper 1.

Further, as shown in FIG. 2, in the upper end portions of the front belt portion 21 and the back belt portion 22, waist elastic strings 41 are arranged along the circumferential direction (lateral direction) of the waist region 221, and the fitting property is enhanced in the waist opening 1a when the diaper 1 is put on. Similarly, leg elastic strings 42 are arranged along the lower end portions of the crotch regions 212 and 222, and the fitting property in the leg opening 1b is enhanced when the diaper 1 is put on.

### Fitting Property of Belt Portions 21 and 22

Subsequently, the fitting property of the front belt portion 21 and the back belt portion 22 when the diaper 1 is put on will be specifically described. It should be noted that in the diaper 1, as illustrated in FIGS. 2 and 3, the front belt portion 21 and the back belt portion 22 have mutually identical structures. Therefore, hereinafter, the front belt portion 21 (hereinafter, also simply referred to as the "belt portion 21") will be mainly described, and the description of the back belt portion 22 will be omitted.

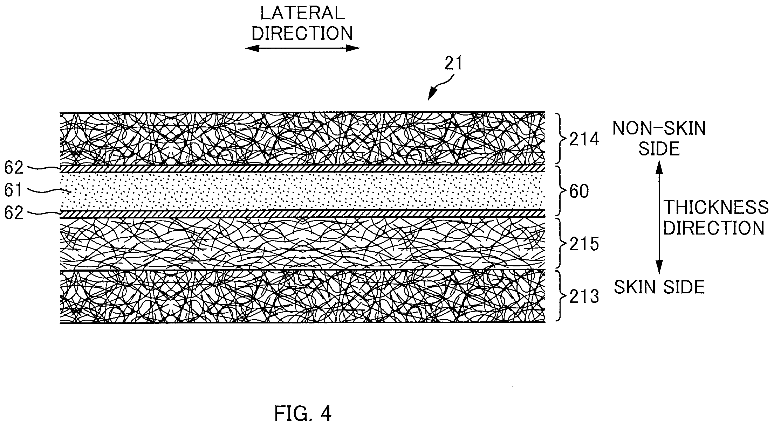

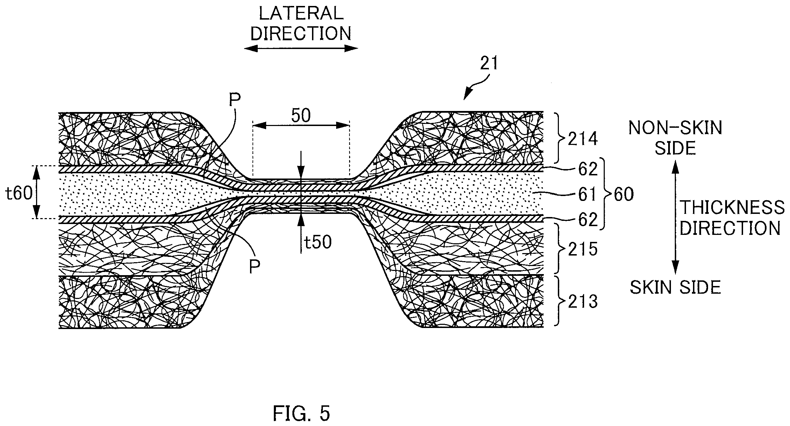

FIG. 4 is an enlarged schematic cross-sectional view of a portion in which the joining portion 50 is not formed, in a thickness-direction cross section of the belt portion 21. FIG. 5 is an enlarged schematic cross-sectional view of a portion in which the joining portion 50 is formed, in the thickness-direction cross section of the belt portion 21. It should be noted that FIGS. 4 and 5 show cross sections of the belt portion 21 in a natural state. The "natural state" refers to a state in which the diaper 1 has been left on its own for a predetermined time. For example, after the diaper 1 in the product state is taken out of the package, the front belt portion 21 and the back belt portion 22 are pulled outward on both lateral sides to put the belt portions 21 and 22 in the "stretched state", the stretched state is maintained for approximately 15 to 30 seconds. Then, the pulling of the diaper 1 is stopped and the diaper is placed on a flat surface such as a table. The state after 5 minutes have elapsed on the flat surface is considered to be the natural state.

As shown in FIG. 4, the belt portion 21 has a structure in which the non-skin-side sheet 214 (exterior sheet), the elastic film 60, the stretchable nonwoven fabric 215, and the skin-side sheet 213 (exterior sheet) are overlaid in this order along the thickness direction starting from the non-skin side toward the skin side. Among these members, the non-skin-side sheet 214, the skin-side sheet 213, and the stretchable nonwoven fabric 215 are so-called nonwoven fabrics and have a predetermined thickness in the thickness direction in a natural state.

On the other hand, the elastic film 60 is a stretchable sheet member having a multilayer structure (in the example in FIG. 4, a three-layer structure) which includes: an elastomer layer 61 having stretchability; and surface layers 62 and 62 that are overlaid and joined on two thickness-direction sides (skin side and non-skin side) of the elastomer layer 61. The elastomer layer 61 is a resin film that is stretchable at least in the lateral direction, and corresponds to the stretchable layer. As the elastomer layer 61, it is possible to use, for example, styrene-based elastomers, olefin-based elastomers, polyester-based elastomers, and other elastomers, and blends thereof. The surface layer 62 is a sheet member having lower stretchability than the elastomer layer and corresponds to the low stretchable layer. As the surface layer 62, it is possible to use, for example, a polyolefin resin (such as polypropylene (PP) or polyethylene (PE)).

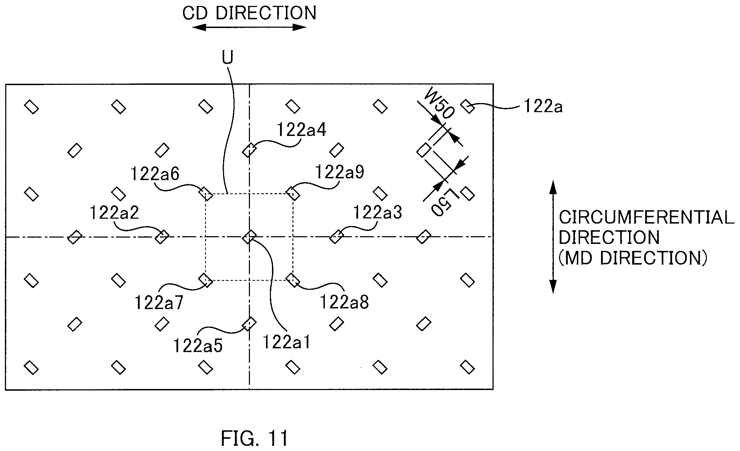

The sheet members 214, 60, 215, and 213 overlaid in the thickness direction are joined to each other by the plurality of joining portions 50. The joining portion 50 in the present embodiment is formed using welding means such as ultrasonic welding or thermal welding, and has a rectangular shape having, for example, a long side length L50 of about 0.5 mm and a short side length W50 of about 0.3 mm (see FIG. 11 below). It should be noted that the shape and size of the joining portion 50 can be appropriately changed.

In a conventional diaper, in the case where the sheet members are joined (welded) to each other by the joining portions 50, the elastic film 60 has thickness-direction through holes formed at locations where the joining portions 50 are provided (for example, see Japanese Patent Application Publication No. 2016-189826). That is, in the lateral direction (stretching/contracting direction), at least a part of the elastic film 60 is in a divided state. In this case, in the portion where the joining portion 50 is formed (a through hole portion formed in the elastic film 60), the stretching/contracting force of the elastic film 60 in the lateral direction is interrupted, causing a risk that the fitting property of the belt portion decreases due to decrease in the stretchability overall.

In contrast, in the present embodiment, in the joining portion 50, at least the surface layer 62 (low stretchable layer) of the elastic film 60 is continuous. That is, the elastic film 60 is not divided in the lateral direction (stretching/contracting direction). Thus, the stretchability of the elastic film 60 in the lateral direction is less likely to decrease, and good fitting property of the belt portion 21 is easily maintained.

On the other hand, compared with the case where the through hole is formed, in the case where no through hole is formed in the elastic film 60 (surface layer 62) in the joining portion 50, there is a risk that the stiffness of the elastic film 60 in the vicinity of the joining portion 50 increases, making the elastic film less likely to be deformed. For example, at the time of wearing the diaper 1, when the wearer moves the body, there is a risk that the elastic film 60 is less likely to follow the movement of the body or a risk that the feeling of the highly stiff joining portion 50 is easily transmitted directly to the wearer's skin.

In contrast, in the diaper 1, as shown in FIG. 5, at an outer edge portion of the joining portion 50, the elastomer layer 61 (stretchable layer) and the surface layer 62 (low stretchable layer) of the elastic film 60 are peeled off, forming a gap P between the two layers. Since the two layers are peeled off, in the vicinity of the joining portion 50, the elastomer layer 61 (stretchable layer) and the surface layer 62 (low stretchable layer) are deformable independently of each other. Thus, even in the case where the wearer moves the body when the diaper 1 is put on, the elastic film 60 is easily deformed in the vicinity of the joining portion 50 according to the movement of the wearer's body. In addition, the gap P is formed in the portion where the elastomer layer 61 (stretchable layer) and the surface layer 62 (low stretchable layer) are peeled off, and the gap P functions as a cushion. The end edge portion (edge) of the joining portion 50 is less likely to be pressed against the wearer's skin. This suppresses the transmission of the feeling of hardness of the joining portion 50 to the wearer's skin, making it possible to enhance the texture.

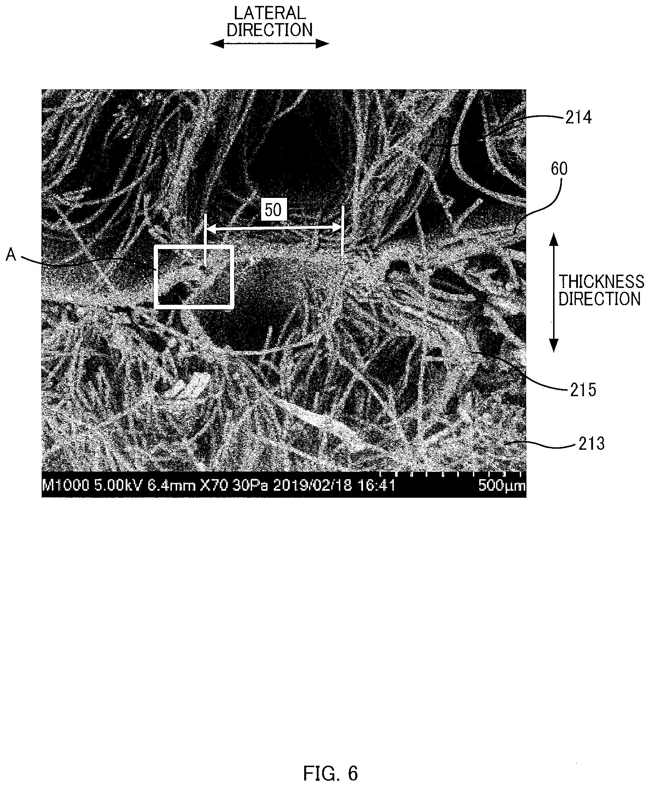

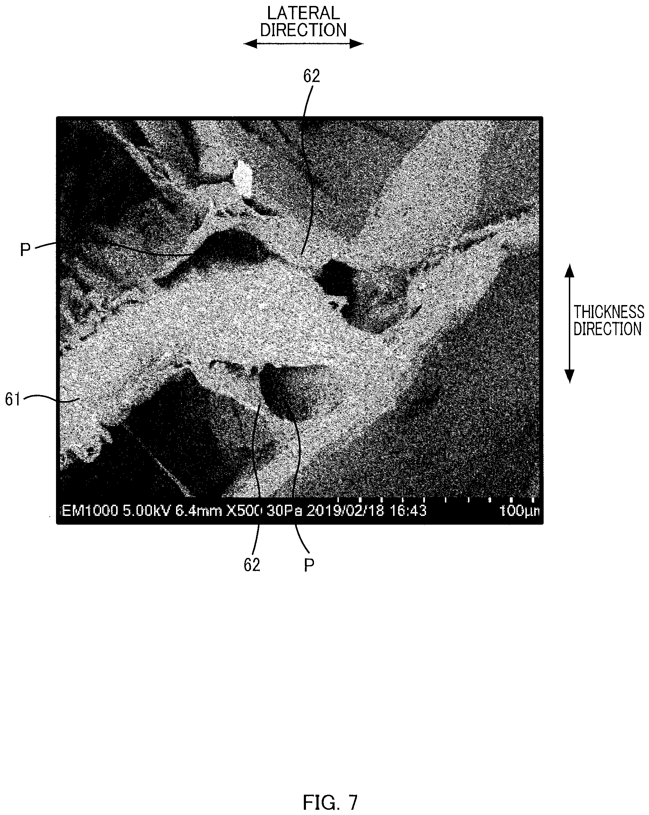

FIG. 6 is a photograph of an example of an actual cross-sectional structure of the belt portion 21 in the thickness direction, and is a diagram illustrating a configuration corresponding to the schematic cross-sectional view of FIG. 5. FIG. 7 is a photograph of a region A in FIG. 6 in an enlarged manner. In FIG. 6, the cross section of the belt portion 21 is magnified 70 times, and in FIG. 7, the cross section of the belt portion 21 is magnified 500 times. It should be noted that in FIG. 6, fibers that constitute the skin-side sheet 213 and the non-skin-side sheet 214 are arranged on two sides (the skin side and the non-skin side) of the joining portion 50 in the thickness direction, and therefore, the configuration in FIG. 6 seems to be different from the configuration in FIG. 5. In other words, the two sides of the joining portion 50 in the thickness direction seem to be covered with the fibers. This is because the photograph in FIG. 6 shows fibers at a position deviated with respect to the joining portion 50 in the vertical direction (a direction corresponding to the depth in FIG. 6) or fibers protruding toward the inner side from two lateral end sides of the joining portion 50. Actually, the configuration in FIG. 6 is equivalent to the configuration in FIG. 5.

FIG. 6 shows that in the joining portion 50 and the outer edge portion thereof, the elastic film 60 (the elastomer layer 61 and the surface layer 62) is continuous and is not discontinuous halfway. Further, FIG. 7 shows that, in the outer edge portion of the joining portion 50, there is a portion where the elastomer layer 61 (stretchable layer) and the surface layer 62 (low stretchable layer) are peeled off from each other, and the gap P is formed in the peeled-off portion.

In this manner, in the belt portion 21 (22) of the diaper 1, the elastic film 60 is provided, which makes it possible to suppress positional deviation during wearing. Further, since at least the surface layer 62 (low stretchable layer) is continuous in the joining portion 50, it is possible to suppress decrease of stretchability. Further, since the portion where the elastomer layer 61 (stretchable layer) and the surface layer 62 (low stretchable layer) are peeled off is provided at the outer edge portion of the joining portion 50, the elastic film 60 can easily follow the movement of the wearer's body, making it possible to enhance the texture. Therefore, good fitting property of the belt portion 21 (22) can be embodied.

In addition, as shown in FIG. 5, in a natural state, the thickness t50 of the belt portion 21 in the central portion (the central portion in the lateral direction and the vertical direction) of the joining portion 50 is smaller than the thickness t60 of the elastic film 60 in a region where the joining portion 50 is not formed (t50 < t60). This is because the sheet members 214, 60, 215, and 213 that constitute the belt portion 21 is welded and compressed in the thickness direction at the joining portion 50, making the joining portion 50 dense. Accordingly, at least in the central portion of the joining portion 50, the elastic film 60 does not exhibit stretchability, and the joining state of the sheet members is stabilized at the joining portion 50, making it easier to maintain the joining strength. Therefore, the exterior sheet (skin side sheet 213 and non-skin-side sheet 214) of the belt portion 21 easily follows the stretching and contraction of the elastic film 60, maintaining good stretchability of the belt portion 21, making it easier to fit the belt portion to the wearer's body.

Further, in the belt portion 21, the skin-side sheet 213 (skin side exterior sheet) is arranged on one side (skin side) of the elastic film 60 in the thickness direction, the non-skin-side sheet 214 (non-skin side exterior sheet) is arranged on the other side (non-skin side) in the thickness direction. The sheet members 213, 60, and 214 are joined in the thickness direction by the joining portion 50. That is, the elastic film 60 is fixed so as to be sandwiched between the exterior sheets (213 and 214) from two sides in the thickness direction. Compared with the case of fixing a surface of the elastic film 60 on only one side in the thickness direction, fixing the surfaces of the elastic film 60 on both sides in the thickness direction increases the joining force between the exterior sheet and the elastic film 60, making it possible to securely fix the exterior sheet and the elastic film 60 even in a small region such as the joining portion 50. Therefore, even in the case where the elastic film 60 stretches and contracts, the exterior sheets (213 and 214) and the elastic film 60 are less likely to peel off, making it possible to improve the stretchability of the entire belt portion 21.

In addition, the elastic film 60 has a structure in which the surface layers 62 and 62 (low stretchable layers) formed of a predetermined resin material are overlaid on two thickness-direction sides (skin side and non-skin side) of the stretchable, elastomer layer 61 (stretchable layer). Therefore, in the case where the joining portion 50 joins the elastic film 60 and the exterior sheets 213 (stretchable nonwoven fabric 215) and 214 in the thickness direction, it is easy to melt and join to each other the resin that constitutes the surface layer 62 and the resin that constitutes the nonwoven fabric of the exterior sheets 213 (215) and 214. That is, by welding the resins on the facing surfaces, it is possible to more firmly join the elastic film 60 and the exterior sheets. Therefore, even in the case where the belt portion 21 greatly stretches and contracts, the elastic film 60 and the exterior sheets are less likely to peel off, making it easier for the belt portion 21 to follow the movement of the wearer's body. Thus, the fitting property of the diaper 1 is enhanced.

Further, in the present embodiment, the surface layer 62 (low stretchable layer) of the elastic film 60 is formed of a polyolefin resin. The exterior sheets 213 (215) and 214 are also formed of fibers containing a polyolefin resin. Thus, when the joining portion 50 joins the surface layer 62 (low stretchable layer) and the exterior sheets 213 (215) and 214 which are overlaid in the thickness direction, the polyolefin resins are easily welded, making it possible to more firmly join the surface layer 62 and the exterior sheets 213 (215) and 214. Therefore, the belt portion 21 easily follows the movement of the wearer's body, and the fitting property of the diaper 1 is further enhanced.

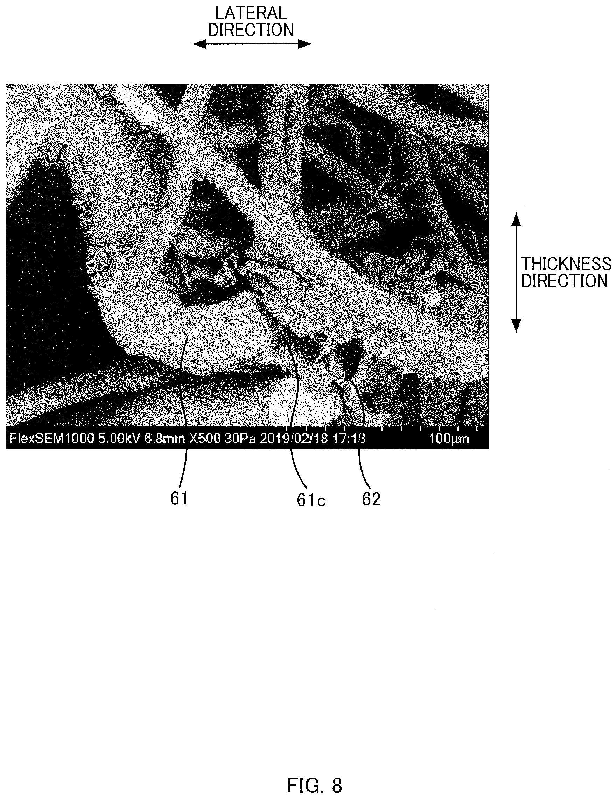

Further, it is described above that, in the belt portion 21 of the diaper 1, at least the surface layer 62 (low stretchable layer) of the elastic film 60 is continuous in the joining portion 50. However, the elastomer layer 61 of the elastic film 60 is not necessarily continuous. FIG. 8 is a photograph of a cross-sectional structure of the belt portion 21 in the thickness direction in the case where a part of the elastomer layer 61 is discontinuous. In the example in FIG. 8, a discontinuous portion 61c is formed in the elastomer layer 61 at the outer edge portion of the joining portion 50. That is, the elastomer layer 61 has a portion that is discontinuous in the lateral direction.

In the case where the discontinuous portion 61c is formed in the elastomer layer 61, there is a risk that the lateral stretching/contracting force of the elastic film 60 decreases in the discontinuous portion 61c. However, since the surface layer 62 of the elastic film 60 is continuous in the diaper 1, the elastic film 60 itself is continuous in the vicinity of the joining portion 50. This prevents the stretching/contracting force of the elastic film 60 from being extremely decreased. On the contrary, the presence of the discontinuous portion 61c in the elastomer layer 61 increases the flexibility of the elastic film 60 in the discontinuous portion 61c. That is, the elastic film 60 is easily flexibly deformed at the outer edge portion of the joining portion 50. Due to the flexible deformation of the elastic film 60 in the vicinity of the highly stiff joining portion 50, it is possible to increase the followability of the belt portion 21 to the movement of the wearer's body when the diaper 1 is put on, and the wearer can be less likely to feel hard touch of the joining portion 50.

Further, in the diaper 1, as for the elastic film 60, as shown in FIG. 2, a plurality of band-like films that are laterally elongated (the circumferential direction of the waist portion 21) are arranged side-by-side spacing in the vertical direction. In other words, in the belt portion 21, the plurality of elastic films 60, 60, . . . are arranged spaced apart from each other in the vertical direction. Since the number of the sheet members that are overlaid in the thickness direction increases in the portion where the elastic film 60 is arranged, there is a risk that the breathability decreases. For example, in the case where the elastic films are continuously arranged across a wide range in the vertical direction, the breathability of the belt portion 21 decreases, and discomfort is more easily caused to the wearer. In contrast, in the diaper 1, by providing a predetermined space between the elastic films 60 and 60 adjacent in the vertical direction, the breathability of the belt portion 21 is easily secured with the space. This can make the wearer be less likely to feel discomfort when the diaper 1 is put on.

Further, the belt portion 21 of the diaper 1 is provided with the stretchable nonwoven fabric 215 that is stretchable at least in the lateral direction. The stretchable nonwoven fabric 215 has a large number of gaps between the entangled fibers, and therefore the stretchable nonwoven fabric 215 has good breathability compared with the elastic film 60. Thus, by providing the stretchable nonwoven fabric 215, the belt portion 21 easily maintains good stretchability and breathability. This makes it to further enhance the fitting property of the belt portion 21 when the diaper 1 is put on, making it possible for the wearer to be less likely to feel discomfort.

Further, it is desirable that the thickness of the surface layer 62 of the elastic film 60 used in the diaper 1 is 0.5% to 8.0% of the thickness of the elastomer layer 61. In the case where the thickness of the surface layer 62 is smaller than 0.5% of the thickness of the elastomer layer 61, the surface layer 62 is more likely to be torn at the time of forming the joining portion 50, and there is a risk that, and the continuity of the surface layer 62 in the joining portion 50 is easily impaired. On the other hand, in the case where the thickness of the surface layer 62 is larger than 8.0% of the thickness of the elastomer layer 61, since the stiffness of the surface layer 62 becomes high, there is a risk that the stretchability of the elastomer layer 61 deteriorates and the stretchability of the belt portion 21 is impaired. Therefore, in the present embodiment, setting the thickness of the surface layer 62 to be 0.5% to 8.0% of the thickness of the elastomer layer 61 makes it possible to enhance the fitting property when the diaper 1 is put on, while maintaining good stretchability of the belt portion 21. It should be noted that, as for the thicknesses of the surface layer 62 and the elastomer layer 61, it is possible to use an average value obtained by capturing a cross-sectional image of the elastic film 60 magnified 500 times or more as shown in FIG. 7 and measuring the thicknesses of a plurality of any points (for example, five points) for each portion (62, 61) in the captured magnified image.

### Method for Manufacturing Diaper 1

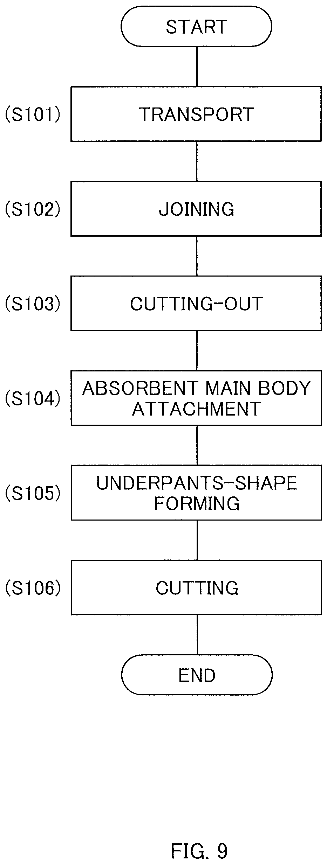

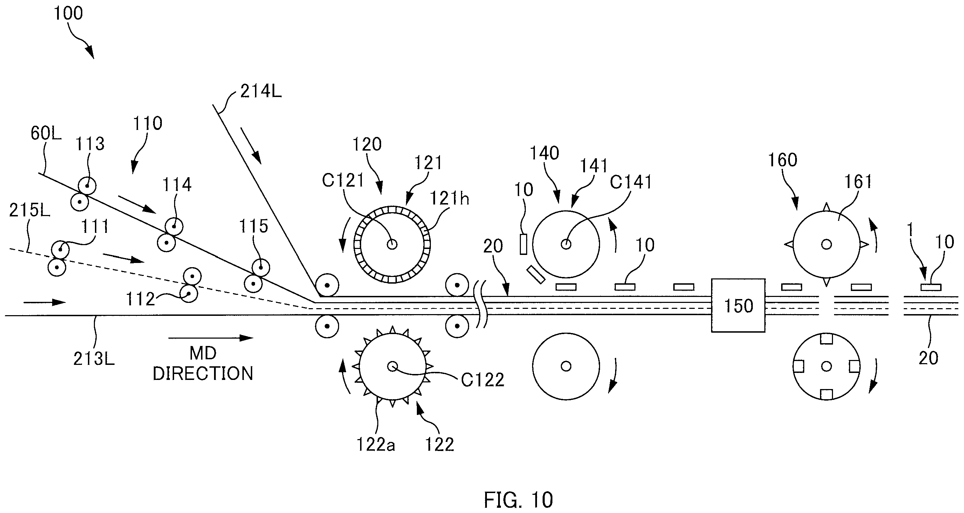

Next, an example of a method for manufacturing the belt portion 21 (22) and the diaper 1 will be described. FIG. 9 is a flow chart of steps in manufacturing the diaper 1. FIG. 10 is a schematic diagram illustrating a manufacturing apparatus 100 for manufacturing the diaper 1. It should be noted that, in FIGS. 9 and 10, in order to facilitate the understanding of the present invention, the absorbent main body 10 is supplied in a completed form, and the description of the method for manufacturing the absorbent main body 10 itself is omitted.

In the manufacturing apparatus 100 shown in FIG. 10, the diaper 1 is continuously manufactured by sequentially performing the steps (S101 to S106) shown in FIG. 9. The manufacturing apparatus 100 includes a transport mechanism 110, a joining mechanism 120, an absorbent-main-body attachment mechanism 140, an underpants-shape forming mechanism 150, and a cutting mechanism 160.

First, there is performed the transport step of transporting various kinds of base materials such as the skin-side sheet 213 that constitutes the front belt portion 21 of the diaper 1, in a predetermined direction of transport (S101). It should be noted that in the manufacturing apparatus 100, the direction of transport is a direction extending along the lateral direction of the diaper 1, and in the following, the direction of transport will also be referred to as an "MD direction". Further, a direction orthogonal to the direction of transport (that is, a direction extending along the vertical direction of the diaper 1) will also be referred to as a "CD direction".

In the transport step, the transport mechanism 110 transports a skin-side-sheet continuous body 213L, a non-skin-side-sheet continuous body 214L, a stretchable-nonwoven-fabric continuous body 215L, and an elastic-film continuous body 60L, at a predetermined transport speed from upstream to downstream in the MD direction. The skin-side-sheet continuous body 213L is in a state where the skin-side sheets 213 are continuous in the MD direction (lateral direction), the non-skin-side-sheet continuous body 214L is in a state where the non-skin-side sheets 214 are continuous in the MD direction (lateral direction), the stretchable-nonwoven-fabric continuous body 215L is in a state where the stretchable nonwoven fabrics 215 are continuous in the MD direction (lateral direction), and the elastic-film continuous body 60L is in a state where the elastic films 60 are continuous in the direction of transport (lateral direction).

At this time, the stretchable-nonwoven-fabric continuous body 215L and the elastic-film continuous body 60L are transported in a state of being stretched in the MD direction at a predetermined stretching ratio (represented by T1), and are joined to the skin-side-sheet continuous body 213L or the like in the joining step (S102) described below. As a result, stretchability along the MD direction (lateral direction) is given to the belt portion 21. The stretching ratio is changed by adjusting the rotation speed of a plurality of nip rollers 111 to 115 provided on the transport path of the transport mechanism 110. For example, as shown in FIG. 10, the nip roller 111 is arranged upstream in the MD direction, and the nip roller 112 is arranged downstream on the path along which the stretchable-nonwoven-fabric continuous body 215L is transported. In this case, by setting the rotation speed of the nip roller 111 to be slower than the rotation speed of the nip roller 112, the stretchable-nonwoven-fabric continuous body 215L can be stretched in the MD direction between the nip roller 111 and the nip roller 112.

Further, in the transport step, the elastic-film continuous body 60L is stretched at a stretching ratio (represented by T2), which is larger than the predetermined stretching ratio T1, then returned to the predetermined stretching ratio T1, and subsequently joined to the skin-side-sheet continuous body 213L or the like. That is, in the belt portion 21 of the diaper 1, the elastic film 60 is once greatly stretched in the MD direction, and then joined to the skin-side sheet 213 or the like after slightly relaxing the stretching. The surface layer 62 that constitutes the elastic film 60 is a member that is less likely to be stretched compared with the elastomer layer 61. Therefore, in the case where the elastic-film continuous body 60L is temporarily put into an excessively stretched state due to application of an impact or the like in the process of transport, there is a risk that the elastomer layer 61 and the surface layer 62 peel off or the surface layer 62 is torn.

Therefore, by greatly stretching the elastic film 60 (particularly, the surface layer 62) in advance in the transport step, sufficient allowance can be provided in the stretchability in the MD direction. Thus, it is possible to prevent the elastomer layer 61 and the surface layer 62 from peeling off, and also it is possible to prevent the surface layer 62 from being torn or punctured in each step (for example, joining step S102). It should be noted that, as in the above-described case, the stretching ratio of the elastic-film continuous body 60L is changed by adjusting the rotation speed of the nip rollers 113 to 115 arranged in the transport path of the elastic-film continuous body 60L in FIG. 10.

Subsequently, there is performed the joining step of joining to each other base materials that have been overlaid in the thickness direction, while being transporting the base materials in the MD direction, forming the exterior body 20 (front side belt portion 21) (S102). In the joining step, ultrasonic vibration and pressure are applied in the thickness direction to each of the overlaid base materials 213, 215, 60, and 214 from the joining mechanism 120, which is provided at a predetermined position in the MD direction, and thus ultrasonic joining is performed. Therefore, a plurality of joining portions 50, 60, . . . are formed on the base materials overlaid in the thickness direction.

The joining mechanism 120 has an ultrasonic roll 121 and an anvil roll 122 in pair provided to face each other in the thickness direction so as to sandwich each base material such as the skin-side-sheet continuous body 213L. The ultrasonic roll 121 and the anvil roll 122 are cylindrical drums that are respectively rotatable around rotation axes C121 and C122 extending along the CD direction. In the thickness direction, the ultrasonic roll 121 is provided on the non-skin-side-sheet continuous body 214L side, and on the outer circumferential surface of the ultrasonic roll 121, a plurality of ultrasonic horns 121h that generate ultrasonic vibrations are provided. On the other hand, the anvil roll 122 is provided on the skin-side-sheet continuous body 213L side, and on the outer circumferential surface of the anvil roll 122, there are provided a plurality of protrusion portions 122a that protrude outward in the radial direction of the anvil roll 122 and that serve as anvils that receive ultrasonic vibration applied by the ultrasonic horn 121h.

It should be noted that in the joining mechanism 120, the ultrasonic horn 121h may be fixed at a predetermined position in the MD direction. That is, the joining step may be performed by causing the fixed ultrasonic horn 121h and the movable anvil roll 122 (protrusion portions 122a) to face each other.

FIG. 11 is a diagram illustrating the arrangement of the plurality of protrusion portions (anvils) 122a provided on the outer circumferential surface of the anvil roll 122. In FIG. 11, the outer circumferential surface of the anvil roll 122 is displayed as a plane in order to simplify the description. As shown in FIG. 11, on the outer circumferential surface of the anvil roll 122, a plurality of protrusion portions 122a, 122a, ... are regularly arranged along the CD direction and the MD direction (circumferential direction). Each of the protrusion portions 122a has a rectangular shape having a long side length L50 of 0.5 mm and a short side length W50 of 0.3 mm. Based on its shape, it is possible to form the rectangular joining portion 50.

In the joining step, the joining portion 50 is formed as follow: while rotating (moving) the ultrasonic roll 121 and the anvil roll 122 at the equal speed to the transport speee at which the base materials (skin side sheet continuous body 213L and the like) are transported in the MD direction, ultrasonic vibration is applied with sandwiching the base materials between the ultrasonic horn 121h and the protrusion portion 122a in the thickness direction. That is, the joining portion 50 is formed while moving the ultrasonic horn 121h and the anvil (protrusion portions 122a) in the same direction at the same speed with respect to the base materials (the elastic-film continuous body 60L and the exterior-sheet continuous bodies 213L and 214L) which are being transported in the MD direction.

In the case where the positions of the ultrasonic horn 121h and the protrusion portion 122a in the MD direction are fixed and ultrasonic vibration is applied to the being-transported base materials from the same position in the MD direction, a difference in relative speed is generated between the base materials to be transported and the protrusion portion 122a and the like. In this case, there is a risk that the protrusion portion 122a and the like act like a brake on the being-transported base materials so that the transport operation of the base materials is hindered or the base materials is torn. In contrast, in the present embodiment, ultrasonic welding is performed while the ultrasonic horn 121h and the protrusion portion 122a move in the same direction at the same speed as the transport speed of the materials with the rotation of the ultrasonic roll 121 and the anvil roll 122. Therefore, the joining portion 50 can be accurately formed without hindering the transport of the materials. Thus, the surface layer 62 (low stretchable layer) of the elastic film 60 is less likely to be torn in the joining portion 50, and a continuous state is easily maintained. This enhances the fitting property of the belt portion 21.

Further, concerning the sheet members (continuous bodies) that pass between the pair of ultrasonic rolls 121 (ultrasonic horns 121h) and the anvil roll 122 (protrusion portions 122a), the number of the sheet members that pass between the elastic film 60 and the anvil roll 122 is larger than the number of the sheet members that pass between the elastic film 60 and the ultrasonic roll 121. In FIG. 10, two sheet members, namely the stretchable-nonwoven-fabric continuous body 215L and the skin-side-sheet continuous body 213L, pass between the elastic film 60 and the anvil roll 122 (protrusion portions 122a). On the other hand, one non-skin-side-sheet continuous body 214L passes between the elastic film 60 and the ultrasonic roll 121 (ultrasonic horn 121h).

In the joining step of the present embodiment, when forming the joining portion 50, the protrusion portion 122a that protrudes outward in the radial direction of the anvil roll 122 is pressed against the elastic film 60, and this causes a risk that an impact acts on the elastic film to tear the elastic film 60. In contrast, by increasing the number of sheet members arranged between the elastic film 60 and the anvil roll 122 (protrusion portions 122a), it is possible to easily relax the impact acting on the elastic film 60. Thus, the elastic film 60 is less likely to be torn, making it easier to maintain the state in which the surface layer 62 (low stretchable layer) of the elastic film 60 is continuous in the joining portion 50. Therefore, the fitting property of the belt portion 21 can be enhanced.

Further, in the manufacturing apparatus 100 of the present embodiment, it is desirable that the area ratio of the protrusion portions 122a on the outer circumferential surface of the anvil roll 122 is 1% to 9%. Here, the "area ratio" means a ratio of the area occupied by the protrusion portions 122a in the surface area of the outer circumferential surface of the anvil roll 122. That is, this means the ratio of the area of the joining portion 50 provided per unit area of the belt portion 21.

The area ratio can be calculated as follows, for example. First, in FIG. 11, the protrusion portion positioned at the center in the MD direction and the CD direction is defined as a protrusion portion 122a1, the protrusion portions arranged on two sides of the protrusion portion 122a1 in the CD direction are defined as protrusion portions 122a2 and 122a3, and the protrusion portions arranged on two sides of the protrusion portion 122a1 in the MD direction are defined as protrusion portions 122a4 and 122a5. There is defined a rectangular region U (a region indicated by a dotted line in FIG. 11) that passes through the intermediate positions respectively located between the protrusion portion 122a1 and the protrusion portions 122a2 to 122a5, which are adjacent to the protrusion portion 122a1 in the MD direction or in the CD direction. This rectangular region U is defined as a unit area of the outer circumferential surface of the anvil roll 122. That is, the outer circumferential surface of the anvil roll 122 corresponds to the plurality of rectangular regions U that are repeatedly arranged side-by-side.

In FIG. 11, the rectangular region U includes the protrusion portions 122a1 and portions of protrusion portions 122a6 to 122a9. Therefore, the area ratio of the protrusion portions 122a on the outer circumferential surface of the anvil roll 122 can be calculated by calculating the area of the rectangular region U and calculating the areas occupied by the whole protrusion portion 122a1 and portions of the protrusion portion 122a6 to 122a9 in the area of the rectangular region U.

In the manufacturing apparatus 100, in the case where the area ratio of the protrusion portions 122a on the outer circumferential surface of the anvil roll 122 is less than 1%, the ratio of the joining portion 50 to the surface area of the belt portion 21 becomes small, causing a risk of weakening the joining strength between the elastic film 60 and the exterior sheets 213 and 214. On the other hand, in the case where the area ratio is more than 9%, the ratio of the joining portion 50 to the surface area of the belt portion 21 becomes large, causing a risk of impairing the stretchability of the elastic film and/or hardening the texture of the belt portion 21. In the present embodiment, by setting the area ratio of the protrusion portions 122a on the outer circumferential surface of the anvil roll 122 to 1% or more and 9% or less, it is possible to suppress deterioration in the stretchability of the elastic film and the texture of the belt portion 21 while sufficiently maintaining the joining strength between the elastic film 60 and the exterior sheets 213 and 214.

Next, returning to FIG. 9, there is performed a cutting-out step (S103) of cutting out the continuous bodies of the joined base materials into a predetermined shape. The cutting-out step can be performed using a cutting-out mechanism (not shown) in FIG. 10. Through this cutting-out step, for example, the crotch region 212 of the belt portion 21 is formed into a trapezoidal shape. It should be noted that, it is also acceptable that in the cutting-out step, by cutting out the continuous bodies of the base materials, the front belt portion 21 (continuous body) and the back belt portion 22 (continuous body) is formed from one base material (continuous body).

After the cutting-out step, there is performed the absorbent-main-body attachment step of forming the continuous body of the exterior body 20 including the front belt portion 21, the back belt portion 22, and the crotch portion 23, and attaching the absorbent main body 10 to the skin side of the exterior body 20 (S104). The absorbent-main-body attachment step is performed by the absorbent-main-body attachment mechanism 140 including the joining drum 141.

The joining drum 141 is a cylindrical drum having a rotation axis C141 extending along the CD direction, and is rotatable around the rotation axis C141 in a state in which the absorbent main body 10 is held on the outer circumferential surface of the joining drum. Then, at the timing when the absorbent main body 10 held on the outer circumferential surface of the joining drum 141 and the continuous body of the exterior body 20 transported in the MD direction come into contact with each other, the holding of the absorbent main body 10 is released, and the absorbent main body 10 is transferred to the skin side surface of the exterior body 20. Thus, the absorbent main body 10 is joined to the continuous body of the exterior body 20.

Subsequently, there is performed an underpants-shape forming step of forming the continuous bodies of the being-transported, various base materials into an underpants shape (S105). In the underpants-shape forming step, the underpants-shape forming mechanism 150 folds the continuous body of the exterior body 20 one time at a fold, which is the substantially center in the CD direction (the longitudinal direction of the absorbent main body 10) (see FIG. 2), and forms the pair of locking portions 24 and 24, shaping the continuous body into an underpants shape, which is continuous in the MD direction.

Finally, there is performed a cutting step of cutting the continuous base material that has formed into an underpants shape, into individual underpants-shaped diapers 1 (S106). The cutting mechanism 160 includes a cutter roll 161 as shown in FIG. 10, and the cutter roll 161 cuts the underpants-shaped diaper that is continuous in the MD direction, at every predetermined length. Thus, each individual underpants-shaped diaper 1 is manufactured. Other Embodiments

The above embodiments are intended to facilitate the understanding of the present invention and are not intended to limit the interpretation of the present invention. The present invention may variously be changed or altered without departing from its gist and encompass equivalents thereof.

### REFERENCE SIGNS LIST

1: diaper (underpants-shaped disposable diaper),
1a: waist opening, 1b: leg opening,
10: absorbent main body, 11: absorbent core,
12: top sheet, 13: back sheet,
20: exterior body,
21: front belt portion, 22: back belt portion,
211, 221: waist region,
212, 222: crotch region,
213, 223: skin-side sheet (exterior sheet), 213L: skin-side-sheet continuous body,
214, 224: non-skin-side sheet (exterior sheet), 214L: non-skin-side-sheet continuous body,
215, 225: stretchable nonwoven fabric, 215 L: stretchable-nonwoven-fabric continuous body,
23: crotch portion, 24: locking portion,
41: waist elastic string, 42: leg elastic string,
50: joining portion (welding portion),
60: elastic film, 60L: elastic-film continuous body,
61: elastomer layer (stretchable layer), 61c: discontinuous portion, 62: surface layer (low stretchable layer),
100: manufacturing apparatus,
110: transport mechanism, 111 to 115: nip roller,
120: joining mechanism,
121: ultrasonic roll, 121h: ultrasonic horn,
122: anvil roll, 122a: protrusion portion (anvil),
140: absorbent-main-body attachment mechanism, 141: joining drum,
150: underpants-shape forming mechanism,
160: cutting mechanism, 161: cutter roll

## Claims

1. An underpants-shaped disposable diaper having a vertical direction, a lateral direction, and a front-back direction that intersect each other,
the disposable diaper comprising:
a belt portion; and
an absorbent main body,
the belt portion having an exterior sheet and an elastic film,
the elastic film being stretchable at least in the lateral direction,
the exterior sheet and the elastic film being joined to each other by a plurality of joining portions,
the elastic film having a stretchable layer and a low stretchable layer,
the stretchable layer being a layer that stretches and contracts in the lateral direction,
the low stretchable layer being a layer that is overlaid on the stretchable layer and has lower stretchability than the stretchable layer,
in a certain joining portion of the plurality of joining portions, the low stretchable layer being continuous,
in at least a part of an outer edge portion of the certain joining portion, the stretchable layer and the low stretchable layer being peeled off.

2. The underpants-shaped disposable diaper according to claim 1, wherein in a natural state,
a thickness of the belt portion in a central portion of the joining portion is smaller than
a thickness of the elastic film in a region where the joining portion is not provided.

3. The underpants-shaped disposable diaper according to claim 1 or 2, wherein the exterior sheet has
a skin-side sheet that is arranged on a skin side with respect to the elastic film, and
a non-skin-side sheet that is arranged on a non-skin side with respect to the elastic film, and
the elastic film, the skin-side sheet, and the non-skin-side sheet are joined by the joining portion.

4. The underpants-shaped disposable diaper according to any one of claims 1 to 3, wherein
the low stretchable layer is formed of a predetermined resin material, and
the low stretchable layer is overlaid on each of two sides of the stretchable layer in a thickness direction.

5. The underpants-shaped disposable diaper according to any one of claims 1 to 4, wherein
the low stretchable layer is formed of a polyolefin resin, and
the exterior sheet is formed of a nonwoven fabric that has fibers containing the polyolefin resin.

6. The underpants-shaped disposable diaper according to any one of claims 1 to 5, wherein
in an outer extension portion of the joining portion, the stretchable layer has a portion that is discontinuous.

7. The underpants-shaped disposable diaper according to any one of claims 1 to 6, wherein
in the belt portion, a plurality of the elastic films are arranged side-by-side with spaces in the vertical direction,
the plurality of elastic films each having a band-like shape and being laterally elongated.

8. The underpants-shaped disposable diaper according to any one of claims 1 to 7, wherein
the belt portion has a stretchable nonwoven fabric that is stretchable at least in the lateral direction.

9. A method for manufacturing an underpants-shaped disposable diaper comprising:
a transport step of transporting an elastic-film continuous body and an exterior-sheet continuous body at a predetermined transport speed in a direction of transport;
a joining step of joining the elastic-film continuous body and the exterior-sheet continuous body, forming a belt portion,
the elastic-film continuous body and the exterior-sheet continuous body being transported in a thickness direction; and
an absorbent-main-body attachment step of attaching an absorbent main body to the belt portion,
in the transport step,
stretching the elastic-film continuous body in the direction of transport at a predetermined stretching ratio,
subsequently loosening the elastic-film continuous body so that the elastic-film continuous body has a stretching ratio lower than the predetermined stretching ratio,
in the joining step, joining the elastic-film continuous body to the exterior-sheet continuous body.

10. The method for manufacturing an underpants-shaped disposable diaper according to claim 9, wherein
in the joining step, the elastic-film continuous body and the exterior-sheet continuous body are joined by applying ultrasonic vibration using an ultrasonic horn and an anvil,
the ultrasonic horn being arranged on a thickness-direction one side of the elastic-film continuous body and the exterior-sheet continuous body,
the anvil being arranged on a thickness-direction other side, and
the ultrasonic horn and the anvil perform the joining while moving in the direction of transport at a speed equal to a transport speed at which the elastic-film continuous body and the exterior-sheet continuous body are transported.

11. The method for manufacturing an underpants-shaped disposable diaper according to claim 10, wherein
the ultrasonic horn is provided on an outer circumferential surface of an ultrasonic roll,
the ultrasonic roll being a cylindrical roll that has a rotation axis extending in a direction orthogonal to the direction of transport,
the anvil is provided on an outer circumferential surface of an anvil roll,
the anvil protrudes outward in a radial direction from the outer circumferential surface of the anvil roll,
the anvil roll being a cylindrical roll that has a rotation axis extending in a direction orthogonal to the direction of transport, and
in the joining step,
a number of sheet members arranged between the elastic film and the anvil roll is larger than
a number of sheet members arranged between the elastic film and the ultrasonic roll.

12. The method for manufacturing an underpants-shaped disposable diaper according to claim 11, wherein
an area ratio of the anvil provided on the outer circumferential surface of the anvil roll is equal to or more than 1% and equal to or less than 9%.
